(19) Europäisches Patentamt
European Patent Office
Office européen des brevets

(11) EP 1 988 393 B1

(12) # FASCICULE DE BREVET EUROPEEN

(45) Date de publication et mention de la délivrance du brevet:
**25.11.2009 Bulletin 2009/48**

(51) Int Cl.:
***G01N 33/24*** *(2006.01)*

(21) Numéro de dépôt: **08290387.3**

(22) Date de dépôt: **17.04.2008**

(54) **Méthode pour déterminer l'origine compositionnelle d'un gaz issu d'une dégradation thermique de matières carbonées**

Methode zur Bestimmung des Ursprungs der Zusammensetzung eines Gases, das aus dem thermischen Abbau von Kohlenstoff hervorgegangen ist

Method for determining the compositional origin of a gas emitted from a thermal breakdown of carbonaceous matter

(84) Etats contractants désignés:
**DE GB**

(30) Priorité: **04.05.2007 FR 0703262**

(43) Date de publication de la demande:
**05.11.2008 Bulletin 2008/45**

(73) Titulaire: **IFP**
**92852 Rueil-Malmaison Cédex (FR)**

(72) Inventeurs:
- **Fusetti, Luc**
  **87920 Condat-sur-Vienne (FR)**
- **Behar, Françoise**
  **75007 Paris (FR)**
- **Lorant, François**
  **94320 Thiais (FR)**

(56) Documents cités:
- **PRINZHOFER A, BATTANI A: "Gas Isotopes Tracing: an Important Tool for Hydrocarbons Exploration" OIL AND GAS SCIENCE AND TECHNOLOGY (REVUE DE L'IFP), vol. 58, no. 2, 2003, pages 299-311, XP002463470**
- **LORANT F ET AL: "Carbon Isotopic and Molecular Constraints on the Formation and the Expulsion of Thermogenic Hydrocarbon Gases" CHEMICAL CEOLOGY, vol. 147, 1998, pages 249-264, XP002463471**
- **BEHAR F, LORANT F: "Thermal Stability of Source Rock and Oil Asphaltenes: Comparison with Kerogen Kinetics in Open system Pyrolysis" IV ALAGO WORKSHOP - BASIN MODELING, 2005, XP002463472**
- **BEHAR F ET AL: "Thermal Evoultion of Crude Oils in Sedimentary Bassins: Experimental Simulation in a Confined System and Kinetic Modeling" mars 1991 (1991-03), REVUE DE L'INSTITUT FRANCAIS DU PETROLE, EDITIONS TECHNIP. PARIS, FR, PAGE(S) 151-181 , XP008078653 ISSN: 1294-4475 * abrégé * * pages 170-173 ***
- **MANGO F, ELROD L: "The Carbon Isotopic Composition of Catalytic Gas: a Comparative Analysis with natural Gas" GEOCHIMICA AND COSMOCHIMICA ACTA, vol. 63, no. 7/8, 1999, pages 1097-1106, XP002463473**

EP 1 988 393 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

Printed by Jouve, 75001 PARIS (FR)

## Description

La présente invention concerne le domaine de l'exploration et la production pétrolière. En particulier l'invention concerne une méthode pour déterminer le type de composés carbonés (huile, kérogène) à l'origine de la genèse, par dégradation thermique, d'un gaz extrait du sous-sol.

La méthode repose sur un modèle de fractionnement isotopique du carbone pour des réactions telles que le craquage thermique de l'huile.

Dans le milieu naturel, la dégradation de la matière sédimentaire en gaz résulte de deux phénomènes, la maturation thermique et la biodégradation. La biodégradation a lieu à de faibles profondeurs, à des températures pour lesquelles les bactéries ne sont pas détruites. Pour un enfouissement de la matière organique plus prononcé, le kérogène peut subir une première dégradation, dite « primaire précoce », qui va entraîner la genèse, en même temps que celle de l'huile, d'un gaz précoce associé. Puis, à la fin de la fenêtre à huile, le kérogène, ainsi que des hydrocarbures générés lors de la phase précédente et retenus dans la roche, vont subir un nouveau craquage thermique produisant un gaz dit « primaire tardif ». Enfin, il est possible que l'huile qui a été expulsée de la roche mère lors du craquage primaire précoce ait continué à s'enfouir. Elle a ainsi pu rencontrer des températures suffisamment élevées pour entraîner sa dégradation en des structures plus petites, dont un gaz dit gaz secondaire. Ainsi, les gaz produits par des opérateurs peuvent avoir cinq origines.

Des phénomènes de migration peuvent entraîner un mélange au sein de la roche réservoir des différents types de gaz précédemment générés, influant sur la quantité et la qualité du gaz en place. L'enjeu lié à la détermination des différentes origines du gaz est donc primordial pour les compagnies pétrolières en terme de prospection au vu des coût très importants engendrés par les politiques d'exploration, particulièrement avec les profondeurs de sédiments rencontrées dans le cas des réservoirs haute pression-haute température. De l'origine des gaz, peut dépendre l'emplacement des forages futurs, leurs profondeurs, ...

## État de la technique

Pour y parvenir, on connaît des méthodes basées sur un outil isotopique permettant de modéliser le fractionnement isotopique du craquage thermique de l'huile. En effet, le fractionnement isotopique du carbone, engendré par la genèse de gaz, n'est pas le même en fonction de sa voie de genèse. La genèse de gaz à partir d'une huile conduit à un important enrichissement isotopique en $^{13}C$ du gaz. Les autres fractions générées ou résiduelles (C6-C14, C15-C20, C20+, résidu) ont des variations de composition isotopique en $^{13}C$ d'un ordre de grandeur beaucoup plus faible et pas forcément unidirectionnelles (pas que de l'enrichissement ou pas que de l'appauvrissement en $^{13}C$)

Les isotopes d'un élément ont des propriétés physiques et chimiques sensiblement différentes. La discrimination entre isotopes au cours des réactions chimiques, biochimiques et des processus physiques constitue le fractionnement isotopique. Le concept de fractionnement isotopique rend compte des variations dans la composition isotopique d'un élément.

L'influence de la dégradation par maturation thermique sur la composition isotopique des gaz générés peut être exprimée comme la résultante de deux effets :

- le premier, appelé effet précurseur (ou terme source), est lié à la distribution des isotopes d'un élément au sein d'une molécule donnée. En effet, dans le cas du carbone, ce dernier possède deux isotopes stables, le carbone 12 et le carbone 13 dont les pourcentages respectifs moyens au sein des molécules organiques naturelles sont de l'ordre de 98.9% et 1.1%. Cependant, il s'agit de pourcentages moyens et, même au sein d'une même molécule naturelle, cette répartition peut varier de façon significative. Il a été montré en particulier que pour les hydrocarbures aromatiques substitués, le carbone 13 se concentre préférentiellement sur les cycles aromatiques plutôt que sur les chaînes hydrocarbonées latérales. En tout état de cause, la composition d'un gaz à la fin de la réaction associée à sa genèse va tendre vers la composition isotope initiale de ses précurseurs au sein de la structure de la (ou les) molécules qui ont subi la dégradation. A cause de l'effet précurseur sus évoqué, cette composition isotopique des précurseurs de gaz est différente de celle de la molécule qui les contient.

- le deuxième effet responsable du fractionnement isotopique, appelé effet cinétique, traduit la différence de vélocité lors de la rupture d'une liaison donnée entre deux atomes lorsque ceux ci sont un isotope particulier de l'élément considéré, dans notre cas le carbone, plutôt qu'un autre.

Le phénomène de fractionnement isotopique peut être modélisé selon deux approches. La première approche raisonne en termes d'équilibre thermodynamique de réactions d'échange entre isotopes d'un même élément. On peut citer par exemple :

James, A.T., 1983. Correlation of natural gas by use of carbon isotopic distribution between hydrocarbon components. American Association of Petroleum Geologists Bulletin 67(7), 1176-1191.

Cependant, cette approche thermodynamique est incapable de rendre compte de faibles variations de $\delta^{13}C$ entre les gaz, sauf à des températures très élevées. Ainsi, il a été montré que la différence calculée entre les $\delta^{13}C$ du propane et du butane est de 1‰ ce que le modèle de James prédit pour des températures en conditions géologiques supérieures à 400°C, ce qui n'est pas réaliste.

La seconde approche étudie l'aspect cinétique d'un tel phénomène qui se traduit par une différence au niveau des vitesses de dégradation instantanées des liaisons $^{12}C$-$^{12}C$ et $^{13}C$-$^{12}C$. En effet, le phénomène de fractionnement isotopique, qui se produit durant des réactions chimiques, tire son origine dans la différence de réactivité en terme cinétique des différents isotopes d'un même élément. Cette différence au niveau des constantes de réaction reflète en particulier une différence au niveau des énergies d'activations associées à ces réactions. Les différents modèles cinétiques de fractionnement isotopique connus peuvent être classés en fonction du formalisme mathématique utilisé pour le calcul des $\delta^{13}C$ et des schémas réactionnels pris en compte.

*Les modèles de distillation*

Ces approches décrivent les fractionnements isotopiques souvent de façon globale par une réaction unique sans considération cinétique proprement dite. Les compositions isotopiques du gaz et de sa source en fonction du degré d'avancement de la réaction suivent explicitement la loi de Rayleigh. Un exemple d'un tel modèle est donné par :

- Berner, U., Faber, E., Scheeder, G., Panten, D., 1995. Primary cracking of algal and land plant kerogens: kinetic modeling of kerogen and oil cracking. Organic Geochemistry 126, 233-245.

Cependant, les modèles basés sur la loi de Rayleigh, ce formalisme supposant le coefficient de fractionnement isotopique indépendant de la température, ne sont pas extrapolables aux conditions géologiques. En effet, les fractionnements isotopiques observés en laboratoire sont beaucoup plus faibles que ceux observés pour des temps de séjour beaucoup plus long et à des températures plus basses dans les conditions géologiques.

*Les modèles cinétiques*

Ce deuxième type d'approche est vraiment une approche cinétique proprement dite au sens où celle ci est basée exclusivement sur une cinétique d'ordre 1. Par rapport à l'approche précédente celle ci présente les avantages d'expliciter les mécanismes réactionnels mis en jeu pour la formation de l'huile et du gaz et, les bilans en $^{12}C$ et $^{13}C$ étant calculés à partir d'équations discrètes, les $\delta^{13}C$ peuvent être simulés à la fois pour la formation et le craquage secondaire des $C_2$-$C_4$. Un exemple d'un tel modèle est donné par :

- Cramer, B., Faber, E., Gerling, P., Krooss, B.M., 2001. Reaction Kinetics of Stable Carbon Isotopes in Natural Gas-Insights from Dry, Open System Pyrolysis Experiments. Energy and Fuels 15, 517-532.

- Tang, Y., Huang, Y., Ellis, G.S., Wang, Y., Kralert, P., Gillaizeau, B., Ma, Q., Hwang, R., 2005. A kinetic model for thermally induced hydrogen and carbon isotope fractionation of individual n-alkanes in crude oil. Geochimica et Cosmochimica Acta 69 (18), 4505-4520.

Une autre méthode est connue pour déterminer l'origine compositionnelle d'un gaz issu d'une dégradation thermique de matières carbonées, dans laquelle on sélectionne au moins un type de matière carbonée susceptible de générer ledit gaz, et l'on construit un modèle de composition isotopique en carbone d'un gaz généré par dégradation thermique dudit type de matière carbonée sélectionné. L'origine compositionnelle dudit gaz est déterminée en comparant la composition isotopique estimée en utilisant le modèle à la composition isotopique mesurée en réalisant des pyrolyses sur des composés modèles du type de matière. Cette méthode est décrite dans:

- Prinzhofer, A., Battani, A., 2003. Gas isotopes tracing: an important tool for hydrocarbons exploration. Oil & Gas Science and Technology - rev. IFP 58(2), 299-311.

*Les modèles statistiques*

Le dernier type d'approche est basée sur des calculs statistiques. Waples et Tornheim ont mis au point un modèle de genèse des gaz hydrocarbures lors du craquage de *n*-alcanes, où les compositions isotopiques du réactif

et des produits résultent d'un traitement statistique des ruptures les liaisons $^{12}C$-$^{12}C$, $^{12}C$-$^{13}C$ et $^{13}C$-$^{13}C$ en fonction du temps.

- Waples, D.W., Tornheim, L., 1978. Mathematical models for petroleum-forming processes: n-paraffins and isoprenoid hydrocarbons. Geochimica Cosmochimica Acta 42, 457-465.

- Waples, D.W., Tornheim, L., 1978. Mathematical models for petroleum-forming processes: carbon isotope fractionation. Geochimica Cosmochimica Acta 42, 467-472.

Cependant, aucun des modèles connus ne permet de déterminer l'origine des gaz formés car aucun de ces modèles ne permet de discriminer les effets précurseur et cinétique du fractionnement isotopique

Un objet de l'invention concerne une méthode pour déterminer le type de composés carbonés (huile, kérogène) à l'origine de la genèse, par dégradation thermique, d'un gaz extrait du sous-sol. Pour y parvenir, la méthode se base sur un outil isotopique permettant de modéliser le fractionnement isotopique ayant lieu lors du craquage d'un mélange carboné tout en discriminant les effets précurseur et cinétique.

**La méthode selon l'invention**

L'invention concerne une méthode pour détermine l'origine compositionnelle d'un gaz *G* issu d'une dégradation thermique de matières carbonées, dans laquelle on sélectionne au moins un type de matière carbonée susceptible de générer ledit gaz *G*, et l'on construit un modèle de composition isotopique en carbone d'un gaz généré *P* par dégradation thermique dudit type de matière carbonée sélectionné. La méthode comporte les étapes suivantes :

- on calibre ledit modèle de composition isotopique en calibrant un effet de cinétique de réaction indépendamment d'un effet précurseur, défini comme l'effet de la composition isotopique en carbone dudit type de matière sur la composition isotopique en carbone dudit gaz généré *P*, en réalisant des pyrolyses sur des composés modèles du type de matière, lesdits composés étant synthétisés et marqués au $^{13}C$ ;

- on estime une composition isotopique en carbone du gaz généré *P*, à l'aide dudit modèle ;

- on mesure une composition isotopique en carbone dudit gaz *G* ; et

- on détermine si le gaz *G* a été généré à partir dudit type de matière carbonée sélectionné, en comparant ladite composition isotopique mesurée à ladite composition isotopique estimée.

Le modèle de composition isotopique en carbone peut être établi en réalisant les étapes suivantes :

- on choisit des composés modèles dudit type de matière carbonée sélectionné ;

- on calibre l'effet cinétique à l'aide de pyrolyses de composés modèles synthétisés et marqués au $^{13}C$ de façon à fixer l'effet précurseur ;

- on calibre l'effet précurseur à l'aide de pyrolyses d'un mélange de composés modèles naturels une fois l'effet cinétique calibré ;

- on étend le modèle à la composition complète dudit type de matière carbonée sélectionné, en supposant que l'un des deux effets, cinétique et précurseur, est le même pour tout autre composé dudit mélange ; et

- on extrapole ledit modèle aux conditions géologiques.

Selon la méthode on peut évaluer la qualité d'un gaz extrait d'une formation souterraine : on sélectionne une huile et un kérogène comme types de matière carbonée, et l'on détermine un modèle de composition isotopique en carbone d'un gaz issu de l'huile, et un modèle de composition isotopique en carbone d'un gaz issu du kérogène. Puis on détermine, à l'aide de ces modèles, la qualité du gaz extrait de la formation souterraine, en évaluant des proportions, au sein du gaz extrait, des différents gaz résultant de l'huile et du kérogène.

D'autres caractéristiques et avantages de la méthode selon l'invention, apparaîtront à la lecture de la description ci-après d'exemples non limitatifs de réalisations, en se référant aux figures annexées et décrites ci-après.

**Présentation succincte des figures**

- la figure 1 est un schéma illustrant les étapes de calibration du modèle de fractionnement isotopique de l'invention.
- la figure 2 montre la droite ln(*k*) en fonction de (1/*T*) dont la pente est (-*Ea*/*R*) et l'ordonnée à l'origine est ln(*A*).
- la figure 3 montre des mesures de production d'éthane en fonction du temps, pour une température de 450°C.
- la figure 4 montre l'ajustement d'une courbe du type $y = \alpha[1-\exp(-x)]$ pour estimer au mieux les observations de la figure 3, et ainsi déterminer *k*.
- la figure 5 montre des mesures de $\delta^{13}C$ en fonction du temps, pour une température de 450°C.
- la figure 6 montre l'ajustement d'un modèle de fractionnement pour estimer au mieux les observations de la figure 5, et ainsi déterminer $\Delta E$ et $\delta^{13}C_{P\infty}$.

**Description détaillée de la méthode**

La méthode selon l'invention permet d'évaluer la quantité et la qualité d'un gaz présent dans une formation souterraine lorsque ce gaz est issu de ruptures de liaisons entre atomes de carbone, suite à une dégradation thermique de composés carbonés par exemple. La figure 1 est un schéma illustrant les étapes de la méthode.

La méthode comporte la définition d'un modèle de variation de la répartition des isotopes du carbone au cours de la dégradation. Puis on calibre ce modèle en traitant de façon indépendante l'effet précurseur et l'effet cinétique de fractionnement isotopique lors de la dégradation thermique. Ce modèle est ensuite exploité pour comparer des mesures de fractionnement isotopique réalisées sur un gaz, de façon à évaluer la quantité et la qualité de ce gaz extrait d'une formation souterraine.

La calibration indépendante de l'effet précurseur et de l'effet cinétique de fractionnement isotopique repose sur la synthèse de composés combinée à un marquage isotopique. Soit un réactif *H*, produisant un produit gazeux *P*. La méthode de calibration comporte les étapes suivantes :

a) choix de composés modèles du réactif ;

b) calibration de l'effet cinétique à l'aide de pyrolyses des composés modèles synthétisés et marqués au $^{13}C$ de façon à fixer l'effet précurseur ;

c) calibration de l'effet précurseur à l'aide de pyrolyse d'un mélange de composés modèles naturels une fois l'effet cinétique calibré.

1- Construction d'un modèle de fractionnement isotopique du carbone

*Principe*

On souhaite établir un modèle de fractionnement isotopique du carbone lorsque ce fractionnement est dû à la production d'un gaz *P* suite à une dégradation thermique d'un réactif *H*. Un modèle de fractionnement isotopique permet d'estimer la variation de la répartition des isotopes du carbone au cours de la dégradation thermique du réactif. On souhaite donc établir un modèle de la composition isotopique du produit *P* au cours du temps. La composition isotopique du produit *P* est notée $\delta^{13}C_P$, et se calcule de la façon suivant :

$$\delta^{13}C_P = \left(R_{C_P} / R_{C_{std}} - 1\right) * 1000$$

avec :

$R_{C_P}$ : rapport isotopique massique $^{13}C/^{12}C$ du produit *P*

$R_{C_{std}}$ : rapport isotopique massique $^{13}C/^{12}C$ du standard PDB

*Définition*

On considère un fractionnement selon le schéma suivant : le produit *P* est généré à partir de la rupture d'une liaison $^{12}C$-$^{12}C$ ou $^{12}C$-$^{13}C$. La réaction s'écrit :

$$R \rightarrow p.^{13}P + ...$$

$$R \rightarrow p.^{12}P + ...$$

avec:

$^{13}P$ le produit *P* issu de rupture de liaisons $^{12}C$-$^{13}C$
$^{12}P$ le produit *P* issu de rupture de liaisons $^{12}C$-$^{12}C$

On considère ensuite que les réactions de production de $^{13}P$ et $^{12}P$ suivent la même loi de vitesse d'ordre 1 que la réaction de production du produit *P*.

La vitesse d'une réaction chimique traduit la variation des concentrations des réactifs *H* ou des produits *P* avec le temps *t*. Pour une réaction de type $H \rightarrow p.P + ...$, la vitesse de disparition du réactif *H* ou celle d'apparition du produit *P* est exprimée par :

$$V = -d[H]/dt = (1/p).d[P]/dt = k.[H]^n$$

avec :

*k* : constante de vitesse ne dépendant que de la température *T*
n : ordre courant de la réaction.

De nombreuses études sur différents composés montre que l'ordre 1 convient pour décrire la réalité. On en déduit la quantité $Y_{P/H_0}$ de produit *P* généré en fonction du temps *t* (en mg par g de charge initiale $H_o$) :

$$Y_{P/H_0}(t) = Y_\infty.[1 - \exp(-k.t)]$$

avec : $Y_\infty$ la valeur vers laquelle *Y* tend en fin de réaction.

En réalisant ces expériences pour différentes températures, on vérifie que *k*(*T*) suit la dépendance en température de la loi d'Arrhenius. On peut ainsi écrire :

$$k(T) = A.\exp(-Ea/R.T)$$

avec :

*A* : facteur de fréquence ou pré exponentiel (en $s^{-1}$ pour une réaction d'ordre 1)
*Ea* : énergie d'activation (en kcal.mol-1)
*R* : constante de gaz parfait (0.001987 kcal.mol-1.K-1)
*T* : température absolue (Kelvin)

La courbe ln(*k*) en fonction de (1/*T*) est donc une droite dont la pente est (-*Ea*/*R*) et l'ordonnée à l'origine est ln(*A*), comme l'illustre la figure 2. Les paramètres cinétiques (énergie d'activation *Ea* et facteur de fréquence *A*) pour la genèse de l'éthane sont donc déterminés en répétant les pyrolyses pour différentes températures, et en déterminant *k*.

En appliquant ces lois aux réactions de production de $^{13}P$ et $^{12}P$, on obtient :

$$\delta^{13}C_P = \left[\left(\delta^{13}C_{P\infty}/1000+1\right)*\left(1-\exp\left(-\alpha.k.t\right)\right)/\left(1-\exp\left(-k.t\right)\right)-1\right]*1000$$

avec :

$\delta^{13}C_{P\infty}$ : composition isotopique du produit *P* en fin de réaction

$\alpha$ : coefficient de fractionnement isotopique

Le coefficient de fractionnement isotopique a pour expression :

$$\alpha = {}^{13}k/{}^{12}k = {}^{13}A/{}^{12}A.\exp\left(-\Delta E/R.T\right)$$

avec : $^{12}k$ et $^{13}k$ les constantes de vitesses respectives des réactions de rupture des liaisons $^{12}C$-$^{12}C$ et $^{12}C$-$^{13}C$, $^{12}A$ et $^{13}A$ les facteurs de fréquence associés et $\Delta E$ la différence entre les énergie d'activation associées. On peut fixer le rapport $^{13}A/^{12}A$ à 1.02 (valeur classique).

Au final, le modèle de fractionnement isotopique selon l'invention s'écrit :

$$\delta^{13}C_P = \left[\left(\delta^{13}C_{P\infty}/1000+1\right)*\left(1-\exp\left(-\left(1{,}02.\exp\left(-\Delta E/R.T\right)\right).k.t\right)\right)/\left(1-\exp\left(-k.t\right)\right)-1\right]*1000$$

Les paramètres à calibrer sont donc :

- la constante de vitesse : *k*
- la composition isotopique du produit *P* en fin de réaction : $\delta^{13}C_{P\infty}$
- la différence entre les énergies d'activation associées : $\Delta E$.

*Calibration*

La méthode selon l'invention est décrite dans le cadre de l'exploitation pétrolière, où les pétroliers souhaitent connaître les composés hydrocarbonés à l'origine des gaz qu'ils produisent au travers d'un puits. La réaction étudiée concerne donc la transformation d'une huile en gaz sous l'effet d'une augmentation de température.

a) choix de composés modèles du réactif

L'huile est composée de différentes classes moléculaires. On se restreint à une classe moléculaire instable simple et suffisamment représentative de la composition chimique de l'huile et de son comportement thermique (ici les aromatiques $C_6$-$C_{14}$). Parmi cette classe moléculaire, on choisit des composés individuels, dits composés modèles, que l'on peut acheter dans le commerce sous forme très pure qui sont parmi les plus présents dans la classe en question et qui sont instables eux aussi (on trouve des composés stables dans la classe dite instable globalement).

Des publications récentes démontrent la réactivité supérieure des hydrocarbures aromatiques vis à vis de leurs homologues saturés lors des réactions de craquage thermique en conditions géologiques, il n'est donc pas nécessaire de prendre ces derniers en compte pour la construction du modèle.

D'autre part, une étude cinétique sur le craquage d'une fraction aromatique $C_6$-$C_{14}$ d'une huile de Type-II, présentée dans le document suivant :

- Al Darouich, T., Behar, F., Largeau, 2006. Thermal cracking of the light aromatic fraction of Safaniya crude oil-experimental study and compositional modelling of molecular classes. Organic Geochemistry 37, 1130-1154.

montre que cette gamme de masses moléculaires caractérise les composés présents autres que saturés qui peuvent être identifiés en détail et quantifiés par les méthodes analytiques actuelles, ce qui correspond donc au système de départ recherché pour la construction d'un tel modèle. En effet, au-delà de $C_{15}$, la complexité du mélange est trop importante et la quantification individuelle des espèces présentes n'est pas possible. Parmi les molécules identifiées par cette étude cinétique, dans la coupe aromatique légère de l'huile, huit sont donc sélectionnées pour leur haut degré de représentativité, tant de la composition de l'huile que de la proportion de gaz produit lors du craquage thermique de cette dernière. Elles appartiennent aux fractions moléculaires des méthylaromatiques et alkylaromatiques :

| | | | | | | | |
|---|---|---|---|---|---|---|---|
| 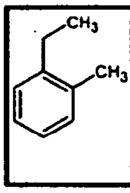 | 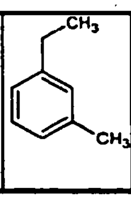 | 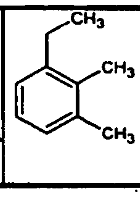 | 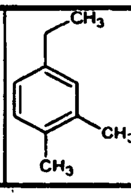 | 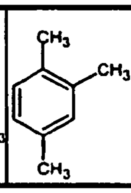 | 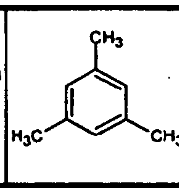 | 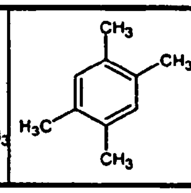 | 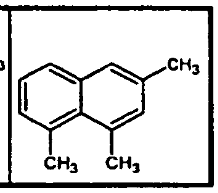 |

Un tel système de composés modèles permet d'être représentatif à plus de 80% du craquage thermique de la fraction aromatique légère de l'huile. Effet, des études ont montré que les benzène, toluène, xylènes et aromatiques soufrés sont stables dans les conditions d'études et non donc pas besoin d'être pris en compte pour cette étude. Seuls les naphténoaromatiques qui contribuent à 18.5% à la fraction légère aromatique d'une huile de Type-II ne sont pas pris en compte à cause de la difficulté à les synthétiser et surtout à les purifier.

b) Calibration de l'effet cinétique

On calibre l'effet cinétique pour chaque composé modèle identifié. Le gaz étudié est, à titre d'exemple, l'éthane. Le composé modèle étudié est, toujours à titre d'exemple, le 2-éthyltoluène. On souhaite établir un modèle de fractionnement isotopique du carbone lorsque ce fractionnement est dû à la production d'éthane par pyrolyse de 2-éthyltoluène.

Pour calibrer les paramètres inconnus du modèle (k, $\delta^{13}C_{P\infty}$, $\Delta E$), on réalise des pyrolyses du réactif (molécule de 2-éthyltoluène) pour différentes températures, suivies d'analyses moléculaires et d'analyses isotopiques.

On réalise également des pyrolyses sur un mélange "artificiel" de ces composés modèles commerciaux afin de quantifier un éventuel effet de mélange sur la cinétique et/ou l'isotopie et dont il faut tenir compte dans la version finale du modèle.

*Analyse moléculaire : détermination de k*

On réalise des pyrolyses de molécule de 2-éthyltoluène (réactif) pour différentes températures et différents temps, puis l'on mesure la quantité d'éthane produit (des analyses moléculaires sont réalisées sur différentes fractions (C1-C4; C6-C14; C15-C20; C20+ et résidu insoluble dans le DCM). Les pyrolyses de la molécule de 2-éthyltoluène suivantes ont ainsi été réalisées :

| T(°C) \ t(h) | 1h | 3h | 6h | 10h | 18h | 24h | 50h | 72h | 120h | 144h | 195h | 216h | 432h | 648h |
|---|---|---|---|---|---|---|---|---|---|---|---|---|---|---|
| 395 | | | | | | | | x | | x | | x | x | x |
| 425 | | x | | x | | | | | x | | | x | | x |
| 450 | x | | | x | | x | x | x | | x | x | | x | |

La croix signifiant une expérience de pyrolyse réalisée pour la température et le temps correspondant.

On en déduit les paramètres cinétiques de la réaction de dégradation du 2-éthyltoluène en éthane, en supposant une réaction d'ordre 1 dont la constante de vitesse *k* suit la dépendance en température de la loi d'Arrhenius.

La constante de vitesse *k* pour la genèse de l'éthane est ainsi calibrée par les données expérimentales. Pour ce faire on mesure la production d'éthane $Y_{P/H_0}$ en fonction du temps *t*, pour une température donnée. La production s'exprime en mg par g de charge initiale $H_0$. La figure 3 montre les mesures réalisées pour une température de 450°C. Puis on ajuste la constante de vitesse *k* de façon à ce qu'une courbe du type $y = \alpha[1-\exp(-x)]$ estime au mieux les observations réalisées (par régression non linéaire par exemple). La figure 4 illustre l'estimation dans le cas de la figure

3. Dans le cadre de la pyrolyse de molécule de 2-éthyltoluène à 450°C, l'estimation donne : $k = 4{,}5923 * 10^{-6}\ s^{-1}$

*Analyse isotopique : détermination de* $\delta^{13}C_{P\infty}$ *et* $\Delta E$

On réalise des pyrolyses de molécule de 2-éthyltoluène (réactif) pour différentes températures. Des analyses isotopiques sont alors réalisées sur différentes fractions ($C_1$-$C_4$; $C_6$-$C_{14}$; $C_{15}$-$C_{20}$; $C_{20+}$ et résidu insoluble dans le DCM). On mesure la composition isotopique du produit $P(\delta^{13}C_P)$. La figure 5 montre les mesures de $\delta^{13}C_P$ réalisées en fonction du temps pour une température de 450°C. Puis on ajuste les paramètres $\delta^{13}C_{P\infty}$ et $\Delta E$ de façon à ce qu'une courbe représentant le modèle de $\delta^{13}C_P$ estime au mieux les observations réalisées (par régression non linéaire par exemple). La figure 6 illustre l'estimation dans le cas de la figure 5. La courbe représente l'équation :

$$\delta^{13}C_P = \left[\left(\delta^{13}C_{P\infty}/1000+1\right) * \left(1-\exp\left(-\left(1{,}02.\exp\left(-\Delta E/R.T\right)\right).k.t\right)\right)/\left(1-\exp\left(-k.t\right)\right)-1\right] * 1000$$

avec: $\delta^{13}C_{P\infty}$ : -28,8 ‰ et $\Delta E$ : 39,6 cal/mol

Cependant, ces valeur de $\delta^{13}C_{P\infty}$ et $\Delta E$ sont obtenues simultanément. $\Delta E$ *est donc* obtenu sans connaître précisément l'effet précurseur, modélisé par $\delta^{13}C_{P\infty}$ : la courbe d'évolution du $\delta^{13}C_P$ en fonction du temps à une forme correctement estimée mais la calibration de l'effet cinétique doit être améliorée dans un premier temps.,

Ce modèle n'est donc pas suffisamment fiable pour discriminer l'origine d'un gaz. Pour améliorer la qualité du modèle. Il faut alors contraindre l'effet précurseur pour modéliser avec la plus grande précision possible l'évolution de la composition isotopique $\delta^{13}C_P$, à savoir l'effet cinétique.

Il faut pouvoir ensuite adapter le modèle à des échantillons naturels. En effet, la courbe d'évolution de la composition isotopique est translatée pour un composé naturel par rapport au composé commercial, car leur histoire synthétique (la façon dont ils se sont formés) est différente (ce qui veut dire que leurs effets précurseurs sont différents, leurs effets cinétiques étant en revanche identiques). Selon l'invention, on contraint l'effet précurseur en réalisant une synthèse de composés modèles au cours de laquelle on implante en des endroits précis de leur squelette carboné, 100% de l'isotope 13 du carbone, greffant ainsi des traceurs géochimiques avant le craquage thermique. En effet, les molécules disponibles commercialement ne permettent pas de connaître à l'avance la composition isotopique des différents groupes qui les constituent car les voies de synthèses sont tenues confidentielles et les intermédiaires non fournis. En revanche, une voie de synthèse comme celle présentée ici permet de résoudre ce problème.

La synthèse mime également « en sens inverse » les étapes de craquage du cycle carboné rencontrées lors de la pyrolyse en associant, pour construire la molécule, des groupes qui seront les mêmes que ceux produits lors du craquage thermique de celle ci. Une telle synthèse permet donc, à condition de connaître le précurseur de chaque gaz, de prédire avec précision la composition isotopique que le gaz atteindra en fin de réaction (l'effet précurseur).

Du 2-éthyltoluène est donc synthétisé. On peut utiliser la réaction suivante :

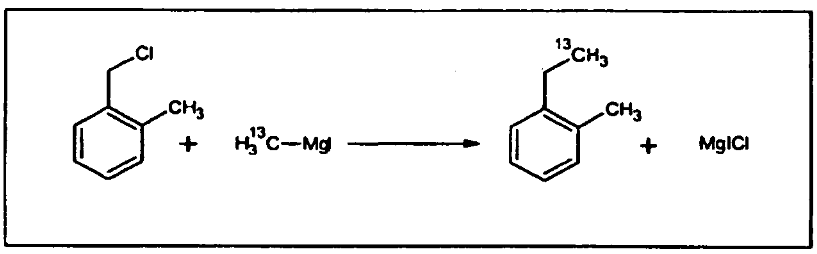

L'effet précurseur $\delta^{13}C_{P\infty}$ étant parfaitement contraint par cette voie de synthèse combinée à un marquage isotopique, l'effet cinétique $\Delta E$ est calibré précisément à l'aide des données isotopiques de pyrolyse de ce composé marqué (on ajuste le $\Delta E$). Ces données sont obtenues comme précédemment décrit : on réalise des pyrolyses des molécules ainsi synthétisées et marquées pour différentes températures, et l'on réalise des analyses isotopiques de façon à déterminer $\Delta E$. En effet, dans le modèle précédent de $\delta^{13}C_p$, $\delta^{13}C_\infty$ est maintenant fixé, et $\alpha$ seul (*via* $\Delta E$) peut être ajusté. Les figures 5 et 6 illustrent un exemple à 450°C.

Les résultats obtenus pour chaque composé modèle marqué sont regroupés pour avoir un modèle de l'effet cinétique de fractionnement isotopique de la fraction aromatique $C_6$-$C_{14}$ : on ajuste tous les $\Delta E$ pour l'ensemble des composés modèles ce qui nous donne une gamme de $\Delta E$, qui constitue un modèle global de la fraction aromatique $C_6$-$C_{14}$. En fait on construit un modèle pour chaque composé et le modèle global est l'ensemble de tous ces modèles.

Afin de vérifier que toutes les données sont suffisamment précises pour permettre l'élaboration d'un tel modèle

on peut compléter les mesures par des bilans massiques et isotopiques

c) calibration de l'effet précurseur

On détermine ensuite précisément l'effet précurseur relatif aux composés naturels qui constituent la fraction $C_6$-$C_{14}$ de l'huile. Pour cela on pyrolyse une coupe aromatique $C_6$-$C_{14}$ de l'huile et en appliquant le modèle précédent obtenu pour les $\Delta E$, on calibre le $\delta^{13}C_\infty$. On considère que pour une molécule fixée, l'effet cinétique de fractionnement isotopique est le même que ce soit une molécule naturelle ou une molécule synthétique commerciale. Seul les effets précurseurs changent. On a ainsi un modèle calibré avec une grande précision et décrivant le fractionnement isotopique des gaz générés lors du craquage thermique de la fraction aromatique $C_6$-$C_{14}$ d'une huile naturelle.

*Extension du modèle à des échantillons naturels*

On étend ensuite le modèle aux autres fractions moléculaires instables de l'huile. On peut déterminer les facteurs de fréquence *A* et les énergies d'activation *Ea* à l'aide de la méthode décrite dans les demandes de brevet suivantes : FR EN/06/08.717 et FR EN/06/08.715.
On suppose ensuite que l'un des deux paramètres $\Delta E$ ou $\delta^{13}C_\infty$, déterminés pour la fraction aromatique $C_6$-$C_{14}$ naturelle, est le même pour chaque nouvelle fraction instable considérée. Si la calibration de l'autre paramètre à l'aide des mesures isotopiques résultant de la pyrolyse d'une telle fraction permet de modéliser correctement les résultats expérimentaux, le résultat est validé. On obtient donc un modèle de fractionnement isotopique du gaz généré par craquage thermique de l'huile mais ceci pour des conditions temps/température rencontrées en laboratoire.

*Extrapolation du modèle aux conditions géologiques*

Enfin, le modèle est extrapolé aux conditions géologiques, c'est à dire pour des conditions de température plus basses (entre 140°C et 220°C dans le cas du craquage secondaire d'une huile) et des temps de séjour beaucoup plus longs (plusieurs millions ou dizaines de millions d'années). Le modèle prédit ainsi le fractionnement isotopique de l'éthane généré par craquage thermique de l'huile dans le réservoir.
Pour cela, les différentes valeurs de $\Delta E$ et de $\delta^{13}C_\infty$ étant connues, on choisit de tels ordres de grandeurs pour *T* et *t* dans les formules de $\alpha$ et de $\delta^{13}C_P$.
De façon très simplifiée, si la variation de $\delta^{13}C_P$ observée pour l'éthane produit par 422h de pyrolyse de 2-éthyltoluène à 450°C est de +7,5‰ alors elle est de + 22,2‰ si le même composé est pyrolysé à 180°C durant 5 millions d'années.

2- Évaluation de la quantité et la qualité d'un gaz issu d'un réservoir

Après avoir été validé sur des échantillons naturels de gaz dont l'origine serait connue, le modèle peut être appliqué à l'étude de cas naturels lors de la prospection gazière et pétrolière. Avec un tel modèle, il devient alors possible par la mesure de la composition isotopique en carbone du gaz se trouvant dans un réservoir, d'en identifier la part qui provient du craquage thermique de l'huile.
Une compagnie pétrolière, après avoir foré un puits à travers un réservoir de gaz, réalise une mesure isotopique en carbone du gaz extrait. L'histoire thermique du bassin étant connue par les géologues de la compagnie, on utilise le modèle de fractionnement isotopique selon l'invention pour déterminer quelle serait la composition d'un gaz secondaire issu du craquage d'une huile qui aurait subie une telle maturation thermique. Pour ce faire, on choisit un type d'huile comme réactif, on choisit des composés modèles, puis l'on calibre le modèle, et on l'applique pour estimer la composition isotopique du gaz $\delta^{13}C_P$ à un temps *t* donné par l'histoire thermique du bassin, si ce gaz provenait exclusivement de l'huile choisie. En comparant cette valeur à la valeur réelle, on en déduit la proportion de gaz secondaire (issu de l'huile) au sein du mélange de gaz présent dans le réservoir. En calibrant le modèle en remplaçant l'huile comme réactif par du kérogène, on établit un modèle pour les gaz primaire précoce et primaire tardif. On détermine alors la proportion de ces gaz. En effet, on un système de deux équations à deux inconnues : la composition isotopique globale du gaz $\delta^{13}C_G$ est mesurée et les compositions isotopiques de chaque gaz ($\delta^{13}C_{G1}$, et $\delta^{13}C_{G2}$) sont modélisées. Soit *x* le pourcentage de gaz primaire et *y* le pourcentage de gaz secondaire. On a une première équation : *x* + *y* = 100. De plus :

$$x*\delta^{13}C_{G1} + y*\delta^{13}C_{G2} = 100*\delta^{13}C_G$$

On peut donc déterminer *x* et *y*. On établit ainsi directement la qualité d'un gaz produit au niveau d'un puits, à savoir

la détermination des proportions des différents gaz résultant de différentes sources.

Enfin, si la quantité des différentes sources est connue par les géologues (quantité de kérogène et/ou d'huile à l'origine) alors le modèle cinétique seul permet de déterminer la quantité de chacun des gaz.

Les informations apportées par de tels modèles couplées à la modélisation de bassin sont d'une grande importance pour l'orientation future du prospect vers une thématique plutôt gaz ou plutôt huile et réduire le risque de puits secs. De l'origine des gaz, peut dépendre l'emplacement des forages futurs, leurs profondeurs, ...

**Applications du modèle de fractionnement isotopique**

Le modèle de fractionnement isotopique selon l'invention peut être appliqué à tous processus engendrant des ruptures différenciées des liaisons C-C en fonction des isotopes de carbone en présence, notamment lorsque le fractionnement isotopique engendré résulte à la fois d'un effet cinétique et d'un effet précurseur.

On peut citer les processus de craquage thermique tel que le raffinage et la combustion, ainsi que les processus bactériologiques de dégradation de l'huile ou du kérogène pour donner (entre autres) du gaz. Tous ces processus engendrent en effet un fractionnement isotopique du carbone qui résulte d'un effet cinétique et d'un effet précurseur:

## Revendications

**1.** Méthode pour déterminer l'origine compositionnelle d'un gaz *G* issu d'une dégradation thermique de matières carbonées, dans laquelle on sélectionne au moins un type de matière carbonée susceptible de générer ledit gaz *G*, et l'on construit un modèle de composition isotopique en carbone d'un gaz généré *P* par dégradation thermique dudit type de matière carbonée sélectionné, **caractérisé en ce que** l'on réalise les étapes suivantes :

- on calibre ledit modèle de composition isotopique en calibrant un effet de cinétique de réaction indépendamment d'un effet précurseur, défini comme l'effet de la composition isotopique en carbone dudit type de matière sur la composition isotopique en carbone dudit gaz généré *P*, en réalisant des pyrolyses sur des composés modèles du type de matière, lesdits composés étant synthétisés et marqués au $^{13}C$ ;
- on estime une composition isotopique en carbone du gaz généré *P*, à l'aide dudit modèle ;
- on mesure une composition isotopique en carbone dudit gaz *G* ; et
- on détermine si le gaz *G* a été généré à partir dudit type de matière carbonée sélectionné, en comparant ladite composition isotopique mesurée à ladite composition isotopique estimée.

**2.** Méthode selon la revendication 1, dans laquelle on établit le modèle de composition isotopique en carbone en réalisant les étapes suivantes :

- on choisit des composés modèles dudit type de matière carbonée sélectionné ;
- on calibre l'effet cinétique à l'aide de pyrolyses de composés modèles synthétisés et marqués au $^{13}C$ de façon à fixer l'effet précurseur ;
- on calibre l'effet précurseur à l'aide de pyrolyses d'un mélange de composés modèles naturels une fois l'effet cinétique calibré ;
- on étend le modèle à la composition complète dudit type de matière carbonée sélectionné, en supposant que l'un des deux effets, cinétique et précurseur, est le même pour tout autre composé dudit mélange ; et
- on extrapole ledit modèle aux conditions géologiques.

**3.** Méthode selon la revendication 1, dans laquelle on sélectionne une huile et un kérogène comme types de matière carbonée, et l'on détermine un modèle de composition isotopique en carbone d'un gaz issu de l'huile, et un modèle de composition isotopique en carbone d'un gaz issu du kérogène, et l'on détermine, à l'aide desdits modèles, la qualité d'un gaz extrait d'une formation souterraine, en évaluant des proportions, au sein dudit gaz extrait, des différents gaz résultant de l'huile et du kérogène.

## Claims

**1.** Method for determining the compositional origin of a gas G from thermal degradation of carbon materials, wherein at least one type of carbon material susceptible to generate said gas G is selected, and a carbon isotopic composition model is built for a gas P generated by thermal degradation of aforesaid type of selected carbon material, **characterized in that** the following steps are performer:

aforesaid model of isotopic composition is calibrated by calibrating a kinetic effect of reaction independently of a precursor effect, defined as the carbon isotopic composition effect of aforesaid material type on the carbon isotopic composition of aforesaid gas P generated, by pyrolyzing model compounds of the material type, said compounds being synthesized and $^{13}C$ labeled;

- a carbon isotopic composition of gas P generated is estimated, using aforesaid model;
- a carbon isotopic composition of aforesaid gas G is measured; and
- it is determined whether gas G has been generated from the selected type of carbon material, by comparing said measured isotopic composition to the estimated isotopic composition.

**2.** Method according to claim 1, wherein the carbon isotopic composition model is established by realizing the following steps:

- choosing model compounds of aforesaid selected carbon material type;
- calibrating the kinetic effect by pyrolyzing synthesized and $^{13}C$ labeled model compounds in order to determine precursor effect;
- calibrating precursor effect by pyrolyzing a mixture of natural model compounds once the kinetic effect calibrated;
- extending the model to the complete composition of said selected carbon material type, assuming that either effects, kinetic or precursor, is the same for any other compound of aforesaid mixture; and
- extrapolating aforesaid model to geological conditions.

**3.** Method according to claim 1, wherein one selects an oil and a kerogen as carbon material types, and one determines a carbon isotopic composition model of a gas derived from oil, and a carbon isotopic composition model of a gas derived from kerogen, and one determines, using aforesaid models, the quality of a gas extracted from a subsoil formation, by assessing the proportions, within said gas extracts, of the various gases resulting from oil and kerogen.

## Patentansprüche

**1.** Verfahren zum Bestimmen des Ursprungs der Zusammensetzung eines Gases *G*, das aus einem thermischen Abbau von kohlenstoffhaltigen Stoffen stammt, wobei mindestens ein kohlenstoffhaltiger Stofftyp ausgewählt wird, der in der Lage ist, das Gas *G* zu erzeugen, und ein Modell der isotopischen Kohlenstoffzusammensetzung eines durch thermischen Abbau des ausgewählten kohlenstoffhaltigen Stofftyps erzeugten Gases *P* konstruiert wird, **dadurch gekennzeichnet, dass** die folgenden Schritte ausgeführt werden:

- Kalibrieren des Modells der isotopischen Zusammensetzung, indem ein kinetischer Effekt der Reaktion unabhängig von einem Vorläufereffekt kalibriert wird, der definiert ist als der Effekt der isotopischen Kohlenstoffzusammensetzung des Stofftyps zur isotopischen Kohlenstoffzusammensetzung des erzeugten Gases *P*, indem Pyrolysen an den Modellverbindungen des Stofftyps ausgeführt werden, wobei die Verbindungen synthetisiert und mit $^{13}C$ markiert werden;
- Bestimmen einer isotopischen Kohlenstoffzusammensetzung des erzeugten Gases *P* mit Hilfe des Modells;
- Messen einer isotopischen Kohlenstoffzusammensetzung des Gases *G*; und
- Bestimmen, ob das Gas *G* ausgehend vom ausgewählten kohlenstoffhaltigen Stofftyp erzeugt wurde, indem die gemessene isotopische Zusammensetzung mit der geschätzten isotopischen Zusammensetzung verglichen wird.

**2.** Verfahren nach Anspruch 1, wobei das Modell der isotopischen Kohlenstoffzusammensetzung erstellt wird, indem die folgenden Schritte ausgeführt werden:

- Wählen der Modellverbindungen des ausgewählten kohlenstoffhaltigen Stofftyps;
- Kalibrieren des kinetischen Effekts mit Hilfe von Pyrolysen von synthetisierten und mit $^{13}C$ markierten Modellverbindungen, um den Vorläufereffekt festzulegen;
- Kalibrieren des Vorläufereffet mit Hilfe von Pyrolysen einer Mischung aus natürlichen Modellverbindungen sobald der kinetische Effekt kalibriert wurde;
- Erweitern des Modells auf die gesamte Zusammensetzung des ausgewählten kohlenstoffhaltigen Stofftyps unter der Annahme, dass einer der beiden Effekte, der kinetische Effekt und der Vorläufereffekt, für jede andere Verbindung der Mischung der gleiche ist; und

- Extrapolieren des Modells auf die geologischen Bedingungen.

**3.** Verfahren nach Anspruch 1, wobei ein Öl und ein Kerogen als kohlenstoffhaltige Stofftypen ausgewählt werden, und ein Modell der isotopischen Kohlenstoffzusammensetzung eines Gases, das aus dem Öl stammt, und ein Modell der isotopischen Kohlenstoffzusammensetzung eines Gases, das aus dem Kerogen stammt, bestimmt wird, und mit Hilfe der Modelle die Qualität eines Gases bestimmt wird, das aus einer unterirdischen Formation extrahiert wird, indem die Anteile der verschiedenen Gase, die aus dem Öl und dem Kerogen hervorgehen, in dem extrahierten Gases evaluiert werden.

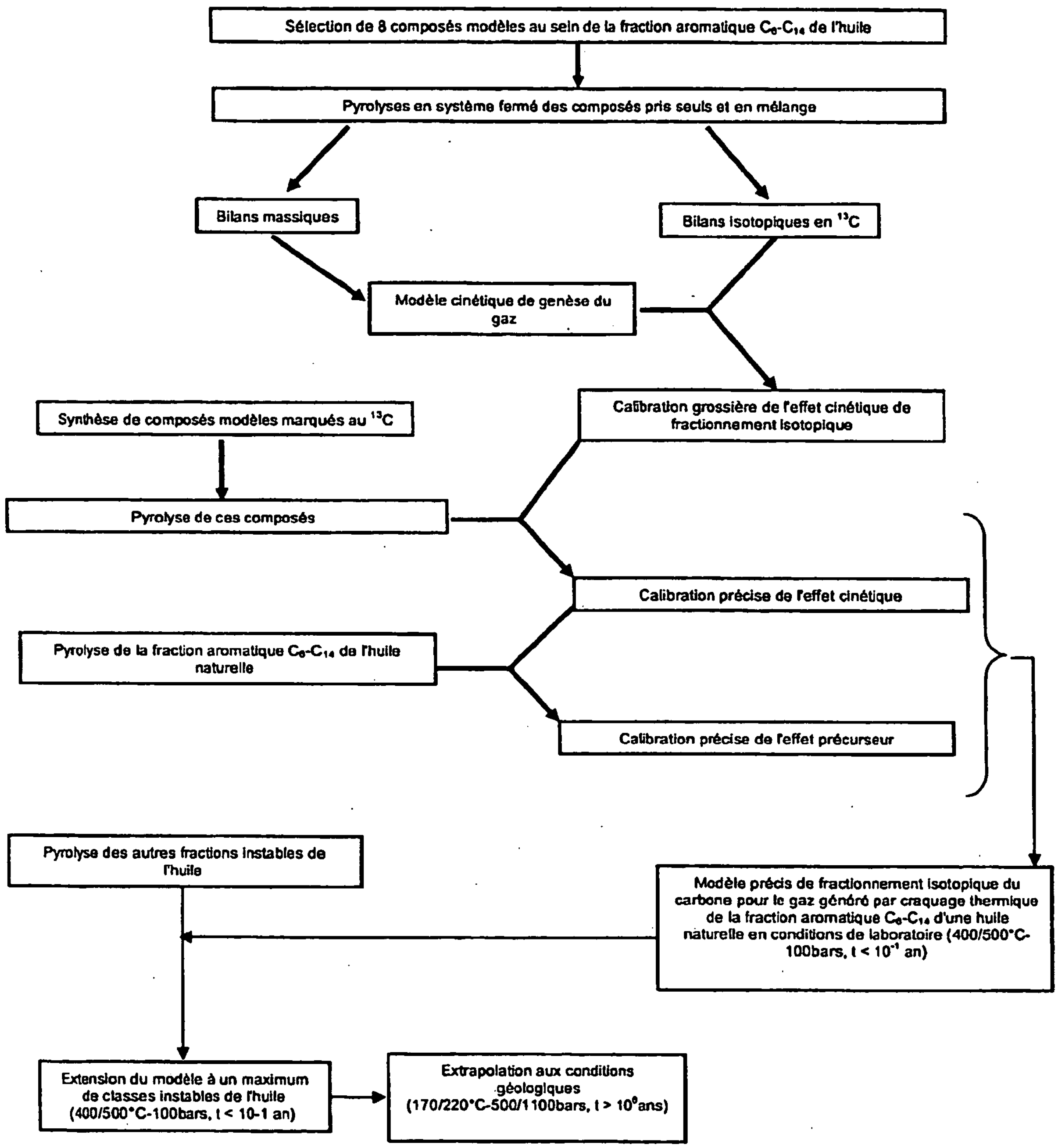

Sélection de 8 composés modèles au sein de la fraction aromatique C6-C14 de l'huile
Pyrolyses en système fermé des composés pris seuls et en mélange
Bilans massiques
Bilans isotopiques en 13C
Modèle cinétique de genèse du gaz
Calibration grossière de l'effet cinétique de fractionnement isotopique
Synthèse de composés modèles marqués au 13C
Pyrolyse de ces composés
Calibration précise de l'effet cinétique
Pyrolyse de la fraction aromatique C6-C14 de l'huile naturelle
Calibration précise de l'effet précurseur
Pyrolyse des autres fractions instables de l'huile
Modèle précis de fractionnement isotopique du carbone pour le gaz généré par craquage thermique de la fraction aromatique C6-C14 d'une huile naturelle en conditions de laboratoire (400/500°C-100bars, t < 10-1 an)
Extension du modèle à un maximum de classes instables de l'huile (400/500°C-100bars, t < 10-1 an)
Extrapolation aux conditions géologiques (170/220°C-500/1100bars, t > 10^6 ans)


Fig. 1

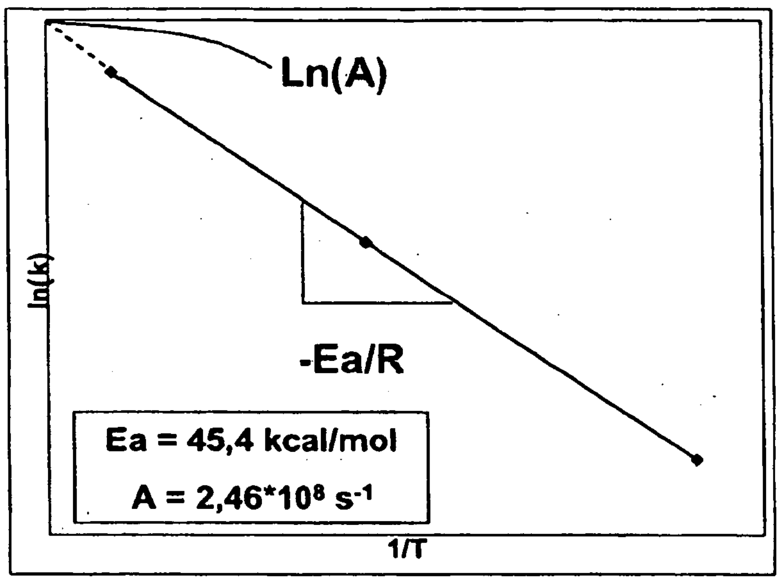


Fig. 2

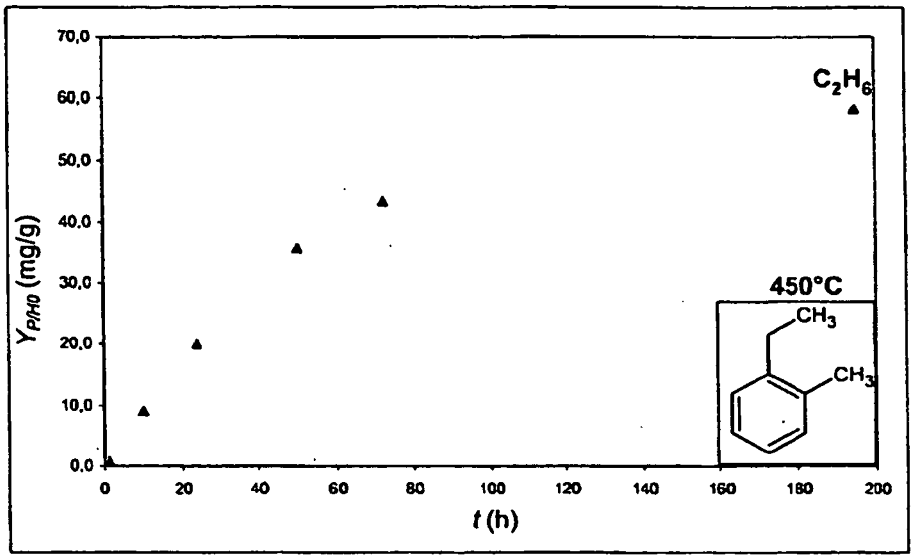
70,0
60,0
50,0
40,0
30,0
20,0
10,0
0,0
0
20
40
60
80
100
120
140
160
180
200
$Y_{P/H0}$ (mg/g)
t (h)
$C_2H_6$
450°C

Fig. 3

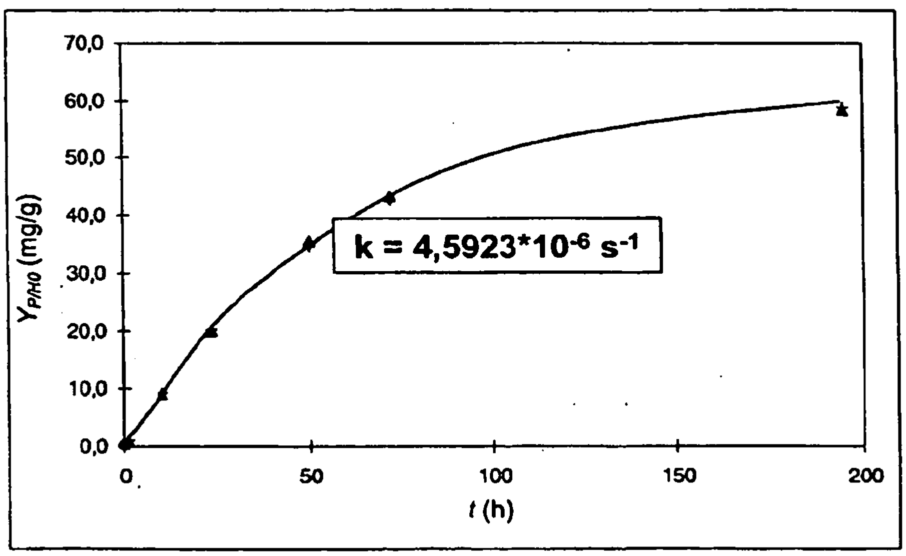
70,0
60,0
50,0
40,0
30,0
20,0
10,0
0,0
0
50
100
150
200
$Y_{P/H0}$ (mg/g)
t (h)
k = 4,5923*10-6 s-1

Fig. 4

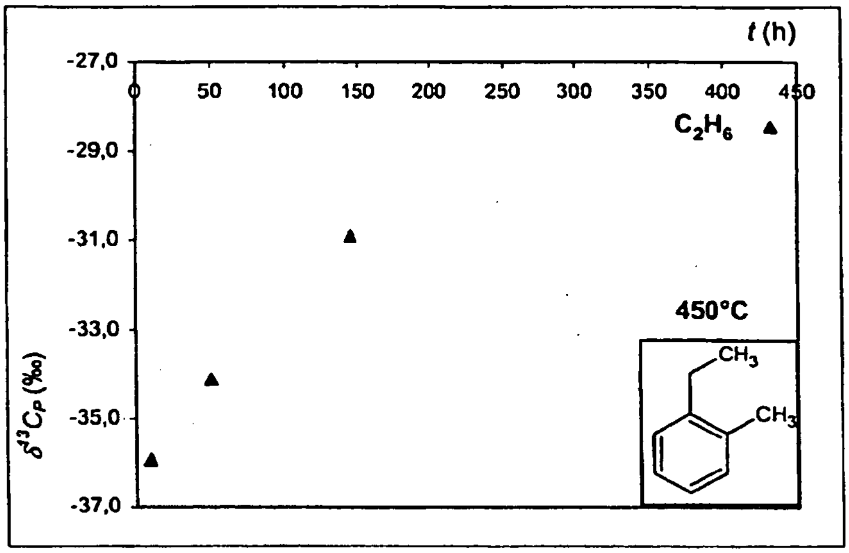
t (h)
-27,0
-29,0
-31,0
-33,0
-35,0
-37,0
0
50
100
150
200
250
300
350
400
450
$C_2H_6$
450°C
$CH_3$
$CH_3$
$\delta^{13}C_P$ (‰)

Fig. 5

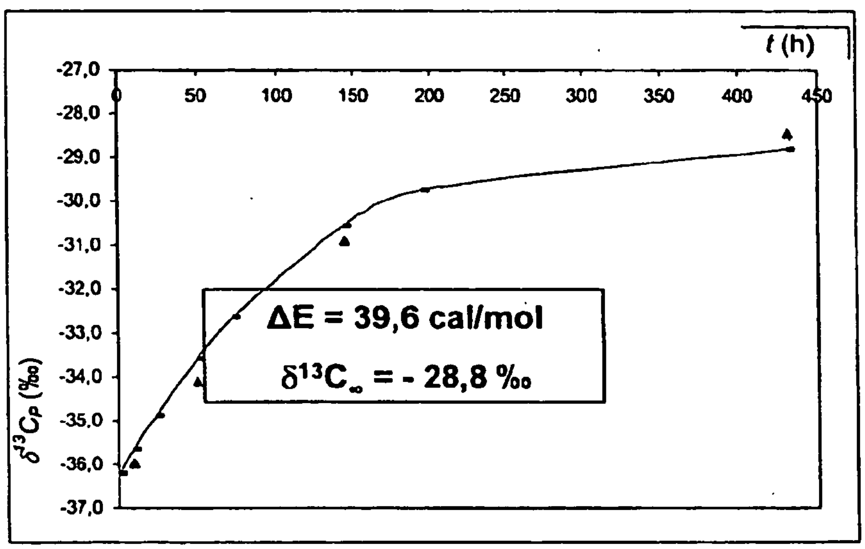
t (h)
-27,0
-28,0
-29,0
-30,0
-31,0
-32,0
-33,0
-34,0
-35,0
-36,0
-37,0
0
50
100
150
200
250
300
350
400
450
ΔE = 39,6 cal/mol
$\delta^{13}C_\infty$ = - 28,8 ‰
$\delta^{13}C_P$ (‰)

Fig. 6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR EN0608717
- FR EN0608715


### Littérature non-brevet citée dans la description


- **James, A.T.** Correlation of natural gas by use of carbon isotopic distribution between hydrocarbon components. *American Association of Petroleum Geologists Bulletin,* 1983, vol. 67 (7), 1176-1191
- **Berner, U. ; Faber, E. ; Scheeder, G. ; Panten, D.** Primary cracking of algal and land plant kerogens: kinetic modeling of kerogen and oil cracking. *Organic Geochemistry,* 1995, vol. 126, 233-245
- **Cramer, B. ; Faber, E. ; Gerling, P. ; Krooss, B.M.** Reaction Kinetics of Stable Carbon Isotopes in Natural Gas-Insights from Dry, Open System Pyrolysis Experiments. *Energy and Fuels,* 2001, vol. 15, 517-532
- **Tang, Y. ; Huang, Y. ; Ellis, G.S. ; Wang, Y. ; Kralert, P. ; Gillaizeau, B. ; Ma, Q. ; Hwang, R.** A kinetic model for thermally induced hydrogen and carbon isotope fractionation of individual n-alkanes in crude oil. *Geochimica et Cosmochimica Acta,* 2005, vol. 69 (18), 4505-4520
- **Prinzhofer, A. ; Battani, A.** Gas isotopes tracing: an important tool for hydrocarbons exploration. *Oil & Gas Science and Technology - rev. IFP,* 2003, vol. 58 (2), 299-311
- **Waples, D.W. ; Tornheim, L.** Mathematical models for petroleum-forming processes: n-paraffins and isoprenoid hydrocarbons. *Geochimica Cosmochimica Acta,* 1978, vol. 42, 457-465
- **Waples, D.W. ; Tornheim, L.** Mathematical models for petroleum-forming processes: carbon isotope fractionation. *Geochimica Cosmochimica Acta,* 1978, vol. 42, 467-472
- **Al Darouich, T. ; Behar, F. ; Largeau.** Thermal cracking of the light aromatic fraction of Safaniya crude oil-experimental study and compositional modelling of molecular classes. *Organic Geochemistry,* 2006, vol. 37, 1130-1154